# EUROPÄISCHE PATENTANMELDUNG

(11) **EP 4 732 747 A2**
(43) Veröffentlichungstag der Anmeldung: **29.04.2026**
(21) Anmeldenummer: 25208021.3
(22) Anmeldetag: 10.10.2025
(51) Int. Cl.: A61B 1/00, A61B 1/005

(54) **SCHLAUCHANORDNUNG FÜR MEDIZINISCHE ANWENDUNGEN UND VERFAHREN ZU IHRER HERSTELLUNG**

(30) Priorität: 28.10.2024 DE 102024131393
(71) Anmelder: Witzenmann GmbH, 75175 Pforzheim (DE)
(72) Erfinder: Breisinger, Michael, 76307 Karlsbad (DE); Heel, Julius, 75173 Pforzheim (DE)
(74) Vertreter: LBP Lemcke, Brommer & Partner Patentanwälte mbB

(57) **Zusammenfassung**

Beschrieben wird eine Schlauchanordnung (1) für medizinische Anwendungen, insbesondere Endoskopie-Schutzschlauch, mit wenigstens einem metallischen Anschlussteil (2), einem metallischen Wickelschlauch (4), einem Geflechtschlauch (5) und einer Knickschutz-Umhüllung - 6), wobei:
der Wickelschlauch (4) mit dem Anschlussteil (2) verbunden ist und das Anschlussteil (2) einen Durchbruch (3) aufweist, der mit einem Innenraum (7) des Wickelschlauchs (4) in Verbindung steht;
der Geflechtschlauch (5) den Wickelschlauch (4) zumindest in einem Abschnitt umhüllt;
das Anschlussteil (2) einen Axialfortsatz (10) aufweist, mit dem es zumindest den Wickelschlauch (4) sowie vorzugsweise auch den Geflechtschlauch (5) axial und mit radialem Abstand (RA) übergreift;
die Knickschutz-Umhüllung (6) den Wickelschlauch (4) sowie vorzugsweise auch den Geflechtschlauch (5) außen vollumfänglich umgibt und sich im Bereich des Anschlussteils (2) sowohl radial innerhalb als auch radial außerhalb des Axialfortsatzes (10) erstreckt.
Außerdem wird ein Verfahren zur Herstellung einer solchen Schlauchanordnung (1) angegeben.

## Beschreibung

Die Erfindung betrifft mit dem Anspruch 1 eine Schlauchanordnung für medizinische Anwendungen, insbesondere einen Endoskopie-Schutzschlauch, mit wenigstens einem metallischen Anschlussteil, einem metallischen Wickelschlauch, einem Geflechtschlauch und einer Knickschutz-Umhüllung.

Die Erfindung betrifft außerdem mit dem Anspruch 11 ein Verfahren zur Herstellung der erfindungsgemäßen Schlauchanordnung.

Solche Schlauchanordnungen sind bekannt. Sie dienen insbesondere zum Schutz von optischen Leitern (Lichtleiterkabel) und ggf. weiteren Leitungselementen, die durch das Anschlussteil und den Wickelschlauch geführt und entsprechend vor Schadeinwirkung von außen geschützt sind. Das Anschlussteil kann zu dienen, die Schlauchanordnung mit einem medizinischen Gerät, z.B. einem Endoskopie-Gerät, zu verbinden. Das Anschlussteil weist bei vorbekannten Schlauchanordnungen der genannten Art einen Axialfortsatz auf, mit dem es den Wickelschlauch sowie vorzugsweise - soweit vorhanden - auch einen Geflechtschlauch axial übergreift. Dieser Axialfortsatz ist regelmäßig in Form einer glattzylindrischen Hülse ausgebildet.

Der genannte Knickschutz besteht bei vorbekannten Schlauchanordnungen der genannten Art regelmäßig aus einer den Wickelschlauch umhüllenden Beschichtung (sog. Schlauchextrude) aus Silikon, auf die im Bereich des Anschlussteils, wo die Knickgefahr am größten ist, ein zusätzliches, separates Formteil (ebenfalls regelmäßig aus Silikon) aufgespritzt ist, das form- bzw. kraftschlüssig außen an dem Anschlussteil gehalten ist, wobei zwischen den beiden Silikonteilen ein Spalt vorhanden ist.

Problematisch bei vorbekannten Schlauchanordnungen der genannten Art ist, dass speziell in der Medizintechnik hohe Hygieneanforderungen bestehen und deshalb entsprechend hohe Anforderungen an die Dichtheit des genannten Spalts zwischen Schlauchextrude und Formteil bestehen. Dieser Spalt ist allerdings in jedem Fall ein potenzieller bzw. nachweislicher Ort für Ablagerungen von Feuchtigkeit, Schmutz, Partikeln oder allgemein Verunreinigungen etc., die sich dort ansammeln und gefährliche Bakteriennester bilden können, zumal die genannte Stelle gar nicht oder nur schwierig und entsprechend zeitaufwändig zu reinigen ist.

Der Erfindung liegt die Aufgabe zugrunde, hier Abhilfe zu schaffen und eine Schlauchanordnung anzugeben, die weniger anfällig für die Ansammlung von Verunreinigungen ist, sich leichter säubern lässt und dennoch einen ausreichenden Knickschutz bietet.

Diese Aufgabe wird erfindungsgemäß gelöst durch eine Schlauchanordnung mit den Merkmalen des Anspruchs 1 sowie durch ein betreffendes Herstellungsverfahren mit den Merkmalen des Anspruchs 11. Vorteilhafte Ausgestaltungen sind in den jeweiligen Unteransprüchen definiert.

Eine erfindungsgemäße Schlauchanordnung für medizinische Anwendungen, insbesondere ein Endoskopie-Schutzschlauch, umfasst wenigstens ein metallisches Anschlussteil, einen metallischen Wickelschlauch, einen Geflechtschlauch und eine Knickschutz-Umhüllung. Der Wickelschlauch und das Anschlussteil bestehen bevorzugt aus (Edel-) Stahl, der Geflechtschlauch kann aus Glasseide bestehen. Der Wickelschlauch ist mit dem Anschlussteil verbunden, und das Anschlussteil weist einen Durchbruch auf, der mit einem Innenraum des Wickelschlauchs in Verbindung steht. Der Geflechtschlauch umhüllt den Wickelschlauch zumindest in einem Abschnitt, vorzugsweise über dessen gesamte Länge. Das Anschlussteil weist einen Axialfortsatz auf, mit dem es zumindest den Wickelschlauch sowie vorzugsweise - soweit vorhanden - auch den Geflechtschlauch mit radialem Abstand axial übergreift. Die Knickschutz-Umhüllung umgibt den Wickelschlauch sowie vorzugsweise - sofern vorhanden - auch den Geflechtschlauch außen vollumfänglich und erstreckt sich im Bereich des Anschlussteils sowohl radial innerhalb als auch radial außerhalb des Axialfortsatzes.

Die Begriffe "axial" und "radial" beziehen sich dabei auf eine Längsachse der Schlauchordnung, wenn diese einen geraden Verlauf aufweist bzw. entsprechend angeordnet ist, wobei "axial" eine Richtung entlang oder parallel zu der Längsachse und "radial" eine Richtung senkrecht dazu bezeichnet.

Ein erfindungsgemäßes Verfahren zur Herstellung der Schlauchanordnung nach einem der vorhergehenden Ansprüche sieht vor, dass
a) optional der Wickelschlauch mit dem Geflechtschlauch umhüllt wird;
b) der Wickelschlauch (und der Geflechtschlauch, sofern vorhanden) mit einer ersten Knickschutz-Umhüllung versehen wird;
c) das Anschlussteil im Bereich seines Axialfortsatzes auf den Wickelschlauch (und den Geflechtschlauch, sofern vorhanden) und die erste Knickschutz-Umhüllung aufgesetzt wird;
d) das Anschlussteil im Bereich seines Axialfortsatzes und die erste Knickschutz-Umhüllung zumindest in einem sich an den Axialfortsatz anschließenden Abschnitt außen mit einer zweiten Knickschutz-Umhüllung versehen werden, die sich mit der ersten Knickschutz-Umhüllung stoffschlüssig zu der Knickschutz-Umhüllung verbindet.

Die Erfindung umfasst bzw. schafft somit eine vollständige stoffschlüssige Verbindung zwischen den Materialien (z.B. Silikonen) der ersten und zweiten Knickschutz-Umhüllungen, also zwischen Schlauchextrude und Formteil, so dass sich eine einstückige, integrale Knickschutz-Umhüllung ergibt, die keinen Spalt mehr aufweist. Dadurch werden Bakteriennester vermieden und die Reinigung vereinfacht, ohne Abstriche beim erreichbaren Knickschutz zu machen.

Bevorzugt ist vorgesehen, dass bei der Herstellung eine vollständige Vernetzung der Silikon-Materialien (oder vergleichbarer Polymere) der ersten und zweiten Knickschutz-Umhüllungen durch eine Verwendung geeigneter Rezepturen und den erfindungsgemäßen Produktionsprozess erst nach dem Aufbringen der zweiten Knickschutz-Umhüllung erfolgt, was dafür sorgt, dass der Spalt zwischen Extrude (erste Knickschutz-Umhüllung) und Formteil (zweite Knickschutz-Umhüllung) geschlossen wird.

Bei einer Ausgestaltung der Erfindung kann durch eine bevorzugte Überarbeitung des Anschlussteils gegenüber seiner vorbekannten Formgebung die Haltbarkeit der Schlauchanordnung deutlich verbessert werden. Speziell können an dem Anschlussteil (im Bereich des Axialfortsatzes) sog. Flanken oder Vorsprünge als Halt für das Polymer-Material (insbesondere Silikon) beim Anspritzprozess vorgesehen sein. Zudem kann das Anschlussteil (im Bereich des Axialfortsatzes) eine Innenkontur als einschneidendes Element zur Montage auf dem mit der ersten Knickschutz-Umhüllung versehenen Wickelschlauch besitzen.

Besondere vorteilhaft ist die Ausbildung einer stoffschlüssigen Verbindung zwischen dem Formteil (zweite Knickschutz-Umhüllung), das bevorzugt nur im Bereich des Anschlussteils und in einem sich axial direkt anschließenden Bereich vorhanden ist, und der Schlauchextrude (erste Knickschutz-Umhüllung), die bevorzugt durchgehend über den gesamten Wickelschlauch vorhanden ist.

Die nachfolgend beschriebenen Ausgestaltungen haben sich in der Praxis als besonders vorteilhaft erwiesen:
Bei einer Weiterbildung der erfindungsgemäßen Schlauchanordnung ist die Knickschutz-Umhüllung aus einem chemisch vernetzbaren (Polymer-) Material, vorzugsweise einem Silikon-Material, gebildet.

Derartige Materialien, insbesondere medizinisches Silikon, haben sich für Anwendungen im Bereich der Medizintechnik etabliert.

Bei Verwendung derartiger Materialien lässt sich die angestrebte einstückige Ausgestaltung der Knickschutz-Umhüllung besonders gut erreichen, dementsprechend eine andere Weiterbildung der erfindungsgemäßen Schlauchanordnung vorsieht, dass die Knickschutz-Umhüllung einstückig ausgebildet ist, was durch entsprechende Vernetzung erreichbar ist. Eine solche Vernetzung sorgt dafür, dass die Knickschutz-Umhüllung, die sich im Bereich des Anschlussteils sowohl radial innerhalb als auch radial außerhalb des Axialfortsatzes erstreckt, zusammen mit der weitergehenden Umhüllung des Wickelschlauchs eine integrale Einheit bildet, wodurch kein verschmutzungsanfälliger Spalt mehr existiert.

Bei wieder einer anderen Weiterbildung der erfindungsgemäßen Schlauchanordnung weist das Anschlussteil an dem Axialfortsatz wenigstens eine Strukturierung seiner äußeren Oberfläche auf.

Diese Strukturierung sorgt für einen besseren Halt des Knickschutz-Materials in dem betreffenden Bereich, was die Haltbarkeit und Widerstandsfähigkeit der Schlauchanordnung verbessert.

Bei einer wieder anderen Weiterbildung der erfindungsgemäßen Schlauchanordnung weist die Strukturierung wenigstens einen ersten umlaufenden radialen Vorsprung auf, vorzugsweise mehrere axial beabstandete erste umlaufende radiale Vorsprünge.

Solche Vorsprünge lassen sich durch bekannte spanende bzw. spanabhebende Herstellungsverfahren leicht und mit flexibler Geometrie ausbilden, was die Herstellung der Schlauchanordnung vereinfacht.

Bei einer entsprechenden Weiterbildung des erfindungsgemäßen Verfahrens wird die äußere Strukturierung durch ein spanendes oder ein spanabhebendes Verfahren hergestellt.

Bei einer noch anderen Weiterbildung der erfindungsgemäßen Schlauchanordnung weist der erste Vorsprung im Längsschnitt eine Sägezahnform auf. Dabei kann vorzugsweise eine steilere Flanke der Sägezahnform, die z.B. unter einem Winkel von 90 Grad bezüglich der Längsachse orientiert ist, einem freien Ende des Anschlussteils, d.h. dem Anschlussende zugewandt sein. Am anderen Ende des Vorsprungs kann der entsprechende Winkel - ohne Beschränkung - z.B. etwa 30 ± 2 Grad bezüglich der Längsachse betragen.

Die Anmelderin hat erkannt, dass sich mit einer solchen Formgebung eine besonders gute Haltbarkeit und Widerstandsfähigkeit der Schlauchanordnung erzielen lässt.

Bei einer vorteilhaften Weiterbildung der erfindungsgemäßen Schlauchanordnung weist das Anschlussteil außen an dem Axialfortsatz wenigstens in einem Bereich eine aufgeraute Oberfläche, eine Riffelung oder eine Rändelung auf. Diese kann vorzugsweise zwischen zwei ersten Vorsprüngen angeordnet sein.

Bei einer entsprechenden Weiterbildung des erfindungsgemäßen Verfahrens wird die aufgeraute Oberfläche durch Sandstrahlen hergestellt.

Die Anmelderin hat erkannt, dass sich auch mit einer solchen Oberflächengestaltung eine besonders gute Haltbarkeit und Widerstandsfähigkeit der Schlauchanordnung erzielen lässt.

Bei einer besonders vorteilhaften Weiterbildung der erfindungsgemäßen Schlauchanordnung weist das Anschlussteil an dem Axialfortsatz wenigstens eine Strukturierung seiner inneren Oberfläche auf.

Mit dieser Strukturierung kann das Anschlussteil mechanisch mit dem Wickelschlauch bzw. mit dessen Umhüllung wechselwirken. Die Anmelderin hat erkannt, dass sich hierdurch die Haltbarkeit und Widerstandsfähigkeit der Schlauchanordnung weiter verbessern lässt.

Bei einer anderen, ganz besonders vorteilhaften Weiterbildung der erfindungsgemäßen Schlauchanordnung weist die Strukturierung (der inneren Oberfläche) wenigstens einen zweiten umlaufenden radialen Vorsprung auf. Vorzugsweise können auch mehrere axial beabstandete zweite umlaufende radiale Vorsprünge vorhanden sein. Der Vorsprung bzw. die Vorsprünge ist/sind schraubengangförmig nach Art wenigstens eines Gewindes ausgebildet.

Mit dieser Strukturierung kann das Anschlussteil auf den Wickelschlauch bzw. auf dessen Umhüllung quasi aufgeschraubt werden. Die Anmelderin hat erkannt, dass sich hierdurch die Haltbarkeit und Widerstandsfähigkeit der Schlauchanordnung noch weiter verbessern lässt.

Dabei kann vorteilhafter Weise vorgesehen sein, dass der zweite Vorsprung in die Knickschutz-Umhüllung einschneidet, was die Haltbarkeit und Widerstandsfähigkeit der Schlauchanordnung zusätzlich weiter verbessert.

Bei einer entsprechenden Weiterbildung des erfindungsgemäßen Verfahrens ist vorgesehen, dass in Schritt c) das Anschlussteil mit seinem Gewinde bzw. dem zweiten Vorsprung auf die erste Knickschutz-Umhüllung aufgeschraubt wird, wobei der zweite Vorsprung in die erste Knickschutz-Umhüllung einschneidet.

Bei einer besonders vorteilhaften Weiterbildung des erfindungsgemäßen Verfahrens ist vorgesehen, dass in Schritt b) als die erste Knickschutz-Umhüllung ein chemisch vernetzbares Material, insbesondere ein Polymer-Material, vorzugsweise ein Silikon-Material, auf den Wickelschlauch aufgebracht wird, vorzugsweise aufextrudiert wird. Des Weiteren wird in Schritt d) die erste Knickschutz-Umhüllung in dem genannten Abschnitt, also einem sich an den Axialfortsatz anschließenden Abschnitt, und das Anschlussteil im Bereich seines Axialfortsatzes mit der zweiten Knickschutz-Umhüllung aus demselben Material oder einem kompatiblen Material versehen, vorzugsweise umspritzt. Dies geschieht, bevor das Material der ersten Knickschutz-Umhüllung vollständig vernetzt ist, damit sich die beiden Knickschutz-Umhüllungen innig zu einer einzigen Knickschutz-Umhüllung verbinden können.

"Kompatibel" bedeutet hierbei, dass sich die beiden Materialien chemisch vernetzen können, um die hiermit verbundenen Vorteile zu erreichen.

Weitere Eigenschaften der Erfindung ergeben sich aus der nachfolgenden Beschreibung von Ausführungsbeispielen anhand der Zeichnung. Dabei bezeichnen gleiche Bezugszeichen gleiche oder gleichwirkende Elemente.

Figur 1 zeigt eine erfindungsgemäße Schlauchanordnung perspektivisch im Längsschnitt; und

Figur 2 zeigt die Schlauchanordnung aus Figur 1 im Längsschnitt und mit zwei Detailvergrößerungen X, Y.

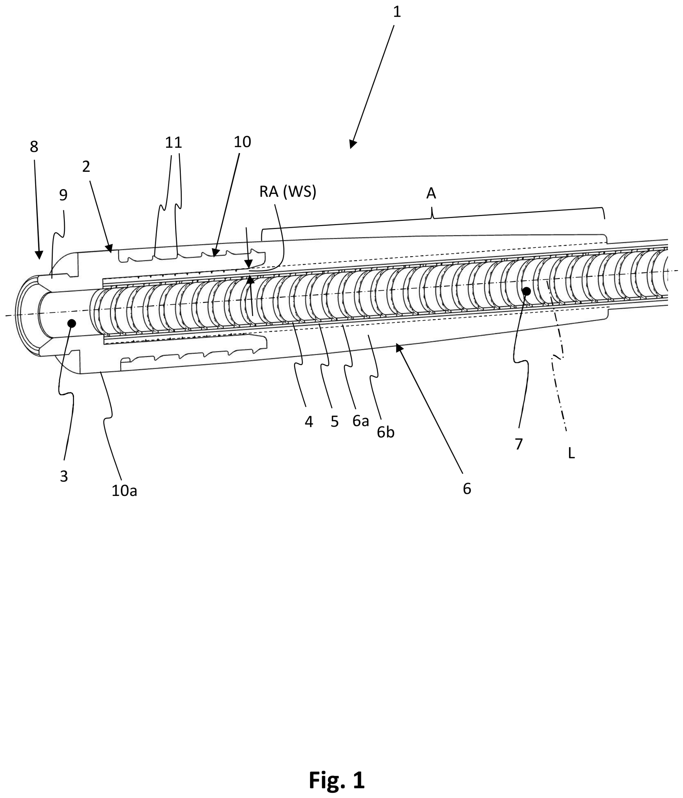

In der Figur 1 ist eine erfindungsgemäße Schlauchanordnung 1 gezeigt. Bezugszeichen L bezeichnet deren Längsachse. Die Schlauchanordnung 1 ist für medizinische Anwendungen geeignet und kann insbesondere als Endoskopie-Schutzschlauch verwendet werden. Sie umfasst ein metallisches Anschlussteil 2, z.B. aus Edelstahl, mit einem zentralen Durchbruch 3.

Sie umfasst weiterhin eine metallischen Wickelschlauch 4, vorzugsweise auch aus Edelstahl, der von einem Geflechtschlauch 5 aus Glasseide (einem seidenähnlichen Material aus Glasfasern) umgeben ist. Der Geflechtschlauch 5 umhüllt den Wickelschlauch 4 über seine gesamte Länge. Des Weiteren umfasst die Schlauchanordnung 1 eine Knickschutz-Umhüllung 6 aus vernetztem (medizinischen) Silikon.

Wie man der Figur 1 entnimmt, ist der Wickelschlauch 4 mit dem Anschlussteil 2 verbunden, wobei das Anschlussteil 2 über seinen Durchbruch 3 mit einem Innenraum 7 des Wickelschlauchs 4 in Verbindung steht, um z.B. Lichtleiterkabel (nicht gezeigt) durch das Anschlussteil 2 in den Wickelschlauch 4 einführen zu können.

An seinem freien Ende 8 weist das Anschlussteil 2 ein (Außen-) Gewinde 9 auf, über das es mit einem medizinischen Gerät, wie einem Endoskopie-Gerät (nicht gezeigt), verbindbar ist.

Das Anschlussteil 2 ist vorzugsweise als Drehteil ausgebildet, wobei ein bei der Herstellung anfallender Spanwurf durch geeignete Reinigung entfernt wurde, insbesondere innen im Bereich des Durchbruchs 3.

Das Anschlussteil 2 weist einen Axialfortsatz 10 auf, mit dem es den Wickelschlauch 4 und den Geflechtschlauch 5 axial und mit radialem Abstand RA, RA > 0, übergreift. Die Knickschutz-Umhüllung 6 umgibt den Wickelschlauch 4 und den Geflechtschlauch 5 außen vollumfänglich sowie über die gesamte Länge der Schlauchanordnung 1 und erstreckt sich dabei im Bereich des Anschlussteils 2, und hier speziell im Bereich des Axialfortsatzes 10, sowohl radial innerhalb (d.h. zwischen Wickelschlauch 4 bzw. Geflechtschlauch 5 und Axialfortsatz 10) als auch radial außerhalb des Axialfortsatzes 10. Dabei bildet die Knickschutz-Umhüllung 6 ein einstückiges Ganzes - ohne Spalt oder dgl. Zwischen Wickelschlauch 4 bzw. Geflechtschlauch 5 und Axialfortsatz 10 beträgt eine Wandstärke WS der Knickschutz-Umhüllung 6 etwa 1 mm, was einem Maß des genannten radialen Abstands RA entspricht. Axial zwischen Axialfortsatz 10 und Gewinde 9 ist ein Radialvorsprung 10a angeordnet.

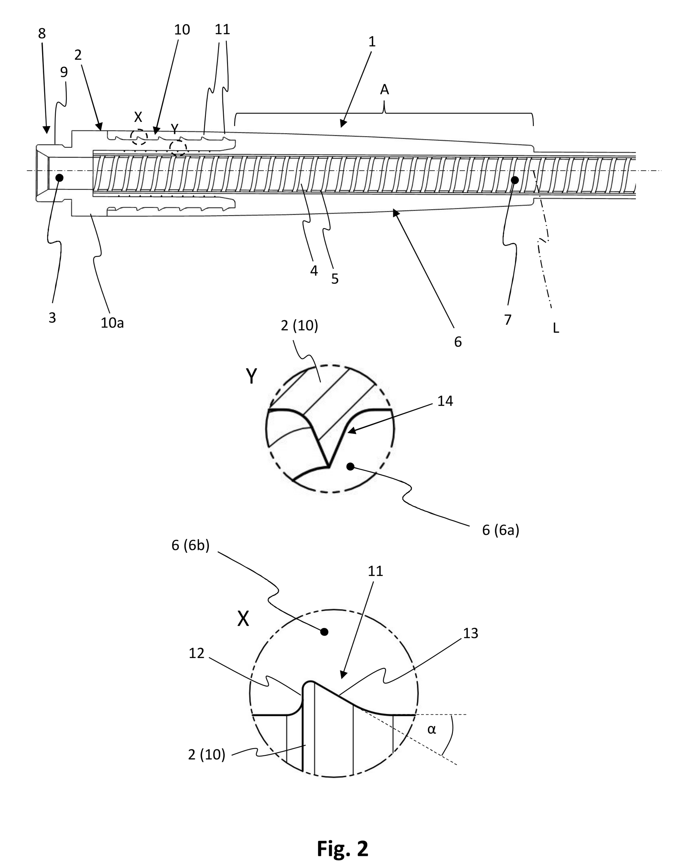

Wie man der Figur 1 noch entnimmt und wie weiter unten anhand von Figur 2 noch genauer erläutert wird, weist das Anschlussteil 2 an dem Axialfortsatz 10 wenigstens eine Strukturierung seiner äußeren Oberfläche auf. Diese Strukturierung umfasst mehrere axial beabstandete erste umlaufende radiale Vorsprünge, die zum Teil mit dem Bezugszeichen 11 bezeichnet sind. Die Vorsprünge 11 weisen im Längsschnitt eine Sägezahnform auf, wobei die steilere Flanke der Sägezahnform dem freien Ende 8 des Anschlussteils 2 zugewandt ist. Dies ist besonders gut im Detailausschnitt X der Figur 2 erkennbar, wo die steilere Flanke der Sägezahnform mit dem Bezugszeichen 12 versehen ist und sich senkrecht zur Längsachse L erstreckt. Der Winkel α zwischen Längsachse L und der anderen (flacheren) Flanke 13 der Sägezahnform beträgt vorliegend etwa 30 Grad.

Das Anschlussteil 2 kann außen an dem Axialfortsatz 10 wenigstens in einem Bereich noch eine (z.B. durch Sandstrahlen) aufgeraute Oberfläche, eine Riffelung oder eine Rändelung aufweisen, was in der Figur nicht zu erkennen ist. Diese zusätzliche Strukturierung kann vorzugsweise zwischen zwei ersten Vorsprüngen 11 angeordnet sein.

Grundsätzlich kommt dabei auch eine Ausgestaltung ganz ohne erste Vorsprünge 11 oder mit einer reduzierten Anzahl an ersten Vorsprüngen 11 in Betracht. In der Figur 1 sind sechs erste Vorsprünge 11 gezeigt. Beispielsweise kann bei einer anderen Ausgestaltung vorgesehen sein, die vier Vorsprünge 11 in der Mitte zu eliminieren und dafür in diesem Bereich eine Rändelung oder eine durch Sandstrahlen veränderte Oberfläche vorzusehen.

Wie man der Figur 1 auch noch entnimmt und wie weiter unten anhand von Figur 2 noch genauer erläutert wird, weist das Anschlussteil 2 an dem Axialfortsatz 10 wenigstens eine Strukturierung seiner inneren Oberfläche auf. Diese Strukturierung umfasst mehrere axial beabstandete zweite umlaufende radiale Vorsprünge 14, die schraubengangförmig nach Art wenigstens eines Gewindes auf der Innenseite des Axialfortsatzes 10 angeordnet sind. Dies ist besonders gut im Detailausschnitt Y der Figur 2 erkennbar. Die zweiten Vorsprünge 14 stehen vorzugsweise um etwa 0,2 bis 0,6 mm radial nach innen gegenüber dem Axialfortsatz 10 hervor.

Beispielsweise kann es sich um drei schraubenförmig gewundenen zweite Vorsprünge 14 handeln, die jeweils drei vollständige Umläufe auf der Innenseite des Axialfortsatzes 10 ausbilden und um ein Drittel ihrer jeweiligen (gleichen) Ganghöhe in der axialen Richtung versetzt angeordnet sind.

Mit den zweiten Vorsprüngen 14 schneidet das Anschlussteil 2 in die Knickschutz-Umhüllung 6 ein. Dadurch kann die Schlauchanordnung 1 Auszugskräften von bis zu 150 N widerstehen.

Bei der Herstellung der Schlauchanordnung 1 kann vorzugsweise wie folgt verfahren werden:
Zuerst wird der Wickelschlauch 4 mit dem Geflechtschlauch 5 in der gewünschten Weise umhüllt. Dann wird der Wickelschlauch 4 mitsamt Geflechtschlauch 5 mit einer ersten Knickschutz-Umhüllung versehen, die Teil der schon erwähnten Knickschutz-Umhüllung 6 ist und die vorliegend aufgrund der bevorzugten Herstellungsart auch als Schlauchextrude bezeichnet wird/wurde. In der Figur 1 ist diese erste Knickschutz-Umhüllung mittels einer gestrichelten Linie symbolisch dargestellt bzw. begrenzt und mit dem Bezugszeichen 6a bezeichnet. Danach wird das Anschlussteil 2 im Bereich seines Axialfortsatzes 10 auf den Wickelschlauch 4 (mit Geflechtschlauch 5) und die erste Knickschutz-Umhüllung 6a aufgesetzt, indem es mit den zweiten Vorsprüngen 14 auf die erste Knickschutz-Umhüllung 6a aufgeschraubt wird, wobei sich die genannten zweiten Vorsprünge 14 in die erste Knickschutz-Umhüllung 6a einschneiden. Anschließend wird das Anschlussteil 2 im Bereich seines Axialfortsatzes 10 und die erste Knickschutz-Umhüllung 6a zumindest in einem sich an den Axialfortsatz 10 anschließenden Abschnitt A (vgl. Figur 2) von außen mit einer zweiten Knickschutz-Umhüllung 6b versehen (vgl. Figur 1), die sich mit der ersten Knickschutz-Umhüllung 6a stoffschlüssig zu einer einzigen, einstückigen Knickschutz-Umhüllung 6 verbindet und sich bis gegen den Radialvorsprung 10a erstreckt. Die ersten Vorsprünge 11 außen an dem Axialfortsatz 10 sorgen für einen festen Halt des Knickschutz-Materials an dem Anschlussteil 2.

Wie schon beschrieben wurde, wird dazu als die erste Knickschutz-Umhüllung 6a ein chemisch vernetzbares Material, insbesondere ein Polymer-Material, vorzugsweise ein Silikon-Material, auf den Wickelschlauch 4 und den Geflechtschlauch 5 aufgebracht, vorzugsweise aufextrudiert. Danach wird die erste Knickschutz-Umhüllung 6a in dem genannten Abschnitt A und das Anschlussteil 2 im Bereich seines Axialfortsatzes 10 mit der zweiten Knickschutz-Umhüllung 6b versehen, die aus demselben Material wie die erste Knickschutz-Umhüllung 6a oder aus einem kompatiblen Material besteht. Das kann durch Umspritzen geschehen und erfolgt zu einem Zeitpunkt, bevor das Material der ersten Knickschutz-Umhüllung 6a vollständig vernetzt ist, damit aus der ersten Knickschutz-Umhüllung 6a und der zweiten Knickschutz-Umhüllung 6b die integrale Knickschutz-Umhüllung 6 entstehen kann.

Am anderen, nicht gezeigten Ende ist die Schlauchanordnung 1 bevorzugt genauso ausgebildet, wie in den Figuren 1 und 2 dargestellt.

## Patentansprüche

1. Schlauchanordnung (1) für medizinische Anwendungen, insbesondere Endoskopie-Schutzschlauch, mit wenigstens einem metallischen Anschlussteil (2), einem metallischen Wickelschlauch (4), einem Geflechtschlauch (5) und einer Knickschutz-Umhüllung -6), wobei:
der Wickelschlauch (4) mit dem Anschlussteil (2) verbunden ist und das Anschlussteil (2) einen Durchbruch (3) aufweist, der mit einem Innenraum (7) des Wickelschlauchs (4) in Verbindung steht;
der Geflechtschlauch (5) den Wickelschlauch (4) zumindest in einem Abschnitt umhüllt;
das Anschlussteil (2) einen Axialfortsatz (10) aufweist, mit dem es zumindest den Wickelschlauch (4) sowie vorzugsweise auch den Geflechtschlauch (5) axial und mit radialem Abstand (RA) übergreift;
die Knickschutz-Umhüllung (6) den Wickelschlauch (4) sowie vorzugsweise auch den Geflechtschlauch (5) außen vollumfänglich umgibt und sich im Bereich des Anschlussteils (2) sowohl radial innerhalb als auch radial außerhalb des Axialfortsatzes (10) erstreckt.

2. Schlauchanordnung (1) nach Anspruch 1, bei der
die Knickschutz-Umhüllung (6) aus einem chemisch vernetzbaren Material, vorzugsweise einem Silikon-Material, gebildet ist.

3. Schlauchanordnung (1) nach Anspruch 1 oder 2, bei der
die Knickschutz-Umhüllung (6) einstückig ausgebildet ist.

4. Schlauchanordnung (1) nach einem der vorhergehenden Ansprüche, bei der
das Anschlussteil (2) an dem Axialfortsatz (10) wenigstens eine Strukturierung seiner äußeren Oberfläche aufweist.

5. Schlauchanordnung (1) nach Anspruch 4, bei der
die Strukturierung wenigstens einen ersten umlaufenden radialen Vorsprung (11) aufweist, vorzugsweise mehrere axial beabstandete erste umlaufende radiale Vorsprünge (11).

6. Schlauchanordnung (1) nach Anspruch 5, bei der
der erste Vorsprung (11) im Längsschnitt eine Sägezahnform aufweist, wobei vorzugsweise eine steilere Flanke (12) der Sägezahnform einem freien Ende (8) des Anschlussteils (2) zugewandt ist.

7. Schlauchanordnung (1) nach einem der Ansprüche 4 bis 6, bei der
das Anschlussteil (2) außen an dem Axialfortsatz (10) wenigstens in einem Bereich eine aufgeraute Oberfläche, eine Riffelung oder eine Rändelung aufweist, vorzugsweise und bei Rückbezug auf Anspruch 5 oder 6 zwischen zwei ersten Vorsprüngen (11).

8. Schlauchanordnung (1) nach einem der vorhergehenden Ansprüche, bei der
das Anschlussteil (2) an dem Axialfortsatz (10) wenigstens eine Strukturierung seiner inneren Oberfläche aufweist.

9. Schlauchanordnung (1) nach Anspruch 8, bei der
die Strukturierung wenigstens einen zweiten umlaufenden radialen Vorsprung (14) aufweist, vorzugsweise mehrere axial beabstandete zweite umlaufende radiale Vorsprünge (14), der/die schraubengangförmig nach Art wenigstens eines Gewindes angeordnet sind.

10. Schlauchanordnung (1) nach Anspruch 9, bei der
der zweite Vorsprung (14) in die Knickschutz-Umhüllung (6) einschneidet.

11. Verfahren zur Herstellung der Schlauchanordnung (1) nach einem der vorhergehenden Ansprüche, bei dem
a) optional der Wickelschlauch (4) mit dem Geflechtschlauch (5) umhüllt wird;
b) der Wickelschlauch (4) mit einer ersten Knickschutz-Umhüllung (6a) versehen wird;
c) das Anschlussteil (2) im Bereich seines Axialfortsatzes (10) auf den Wickelschlauch (4) und die erste Knickschutz-Umhüllung (6a) aufgesetzt wird;
d) das Anschlussteil (2) im Bereich seines Axialfortsatzes (10) und die erste Knickschutz-Umhüllung (6a) zumindest in einem sich an den Axialfortsatz (10) anschließenden Abschnitt (A) außen mit einer zweiten Knickschutz-Umhüllung (6b) versehen werden, die sich mit der ersten Knickschutz-Umhüllung (6a) stoffschlüssig zu der Knickschutz-Umhüllung (6) verbindet.

12. Verfahren nach Anspruch 11 bei Rückbezug auf Anspruch 9, bei dem
in Schritt c) das Anschlussteil (2) mit seinem zweiten Vorsprung (14) auf die erste Knickschutz-Umhüllung (6a) aufgeschraubt wird, wobei der zweite Vorsprung (14) in die erste Knickschutz-Umhüllung (6a) einschneidet.

13. Verfahren nach Anspruch 11 oder 12 bei Rückbezug auf Anspruch 5 oder 6, bei dem die äußere Strukturierung durch ein spanendes Verfahren hergestellt wird.

14. Verfahren nach einem der Ansprüche 11 bis 13 bei Rückbezug auf Anspruch 7, bei dem
die aufgeraute Oberfläche durch Sandstrahlen hergestellt wird.

15. Verfahren nach einem der Ansprüche 11 bis 14, bei dem
in Schritt b) als die erste Knickschutz-Umhüllung (6a) ein chemisch vernetzbares Material, insbesondere Polymer-Material, vorzugsweise Silikon-Material, auf den Wickelschlauch (4) aufgebracht wird, vorzugsweise aufextrudiert wird, und
in Schritt d) die erste Knickschutz-Umhüllung (6a) in dem genannten Abschnitt (A) und das Anschlussteil (2) im Bereich seines Axialfortsatzes (10) mit der zweiten Knickschutz-Umhüllung (6b) aus demselben Material oder einem kompatiblen Material versehen wird, vorzugsweise umspritzt wird, bevor das Material der ersten Knickschutz-Umhüllung (6a) vollständig vernetzt ist.
